(19) Europäisches Patentamt
European Patent Office
Office européen des brevets

(11) Veröffentlichungsnummer: **0 193 065 B1**

(12) # EUROPÄISCHE PATENTSCHRIFT

(45) Veröffentlichungstag der Patentschrift: **23.08.95**

(51) Int. Cl.[6]: **C07D 265/30**, C07C 43/12, C07C 43/17, C07C 43/192, C07C 41/16, A01N 33/08

(21) Anmeldenummer: **86101984.2**

(22) Anmeldetag: **17.02.86**

Die Akte enthält technische Angaben, die nach dem Eingang der Anmeldung eingereicht wurden und die nicht in dieser Patentschrift enthalten sind.

(54) **Morpholinderivate, Verfahren zu ihrer Herstellung und ihre Verwendung als Fungizide.**

(30) Priorität: **22.02.85 DE 3506117**

(43) Veröffentlichungstag der Anmeldung:
**03.09.86 Patentblatt 86/36**

(45) Bekanntmachung des Hinweises auf die Patenterteilung:
**23.08.95 Patentblatt 95/34**

(84) Benannte Vertragsstaaten:
**AT BE CH DE FR GB IT LI NL SE**

(56) Entgegenhaltungen:
**EP-A- 0 129 211**
**EP-A- 0 129 321**
**AT-B- 162 899**
**AT-B- 193 860**
**AT-B- 310 148**
**AT-B- 321 311**
**AT-B- 348 984**
**CH-A- 267 634**
**CH-A- 331 991**
**DE-A- 1 925 697**
**DE-B- 2 332 277**
**GB-A- 655 999**
**GB-A- 1 590 148**
**US-A- 2 105 828**
**US-A- 3 472 845**
**US-A- 3 478 096**
**US-A- 3 840 605**
**US-A- 4 299 957**
**US-A- 4 474 988**

**SOVIET INVENTIONS ILLUSTRATED, Sektion CH, Woche B39, 7. November 1979 DERWENT PUBLICATIONS LTD., London, CO3**

(73) Patentinhaber: **BASF Aktiengesellschaft**
**Carl-Bosch-Strasse 38**
**D-67063 Ludwigshafen (DE)**

(72) Erfinder: **Rentzea, Costin, Dr.**
**Richard-Kuhn-Strasse 1-3**
**D-6900 Heidelberg (DE)**
Erfinder: **Buschmann, Ernst, Dr.**
**Georg-Ludwig-Krebs-Strasse 10**
**D-6700 Ludwigshafen (DE)**
Erfinder: **Goetz, Norbert, Dr.**
**Schoefferstrasse 25**
**D-6520 Worms 1 (DE)**
Erfinder: **Ammermann, Eberhard, Dr.**
**Sachsenstrasse 3**
**D-6700 Ludwigshafen (DE)**
Erfinder: **Pommer, Ernst-Heinrich, Dr.**
**Berliner Platz 7**
**D-6703 Limburgerhof (DE)**

EP 0 193 065 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Amine, Verfahren zu deren Herstellung, fungizide Mittel, die die neuen Wirkstoffe enthalten, Verfahren zur Herstellung solcher fungiziden Mittel sowie Verfahren zur Bekämpfung von Schadpilzen mit diesen Fungiziden.

Es ist bekannt, N-Tridecyl-2,6-dimethylmorpholin bzw. 2,6-trans-Dimethylmorpholinderivate oder Salze dieser Verbindungen (z.B. die Acetate) als Fungizide zu verwenden (vgl. DE-A 1 164 152, DE-A 1 173 722, EP-A 129 211).

Fungizide Morpholinderivate sind weiterhin bekannt aus der EP-A 129 321.

Aus der Patentliteratur ist darüber hinaus eine Reihe von Verbindungen bekannt, welche zwar gemeinsame Strukturmerkmale mit den vorstehend genannten Fungiziden haben, deren Verwendung jedoch nicht auf dem Gebiet des Pflanzenschutzes liegt (vgl. CH-A 331 991, US-A 4 299 957, US-A 3 472 845, US-A 2 105 828, CH-A 267 634, US-A 4 474 988, US-A 3 478 096, AT-B 348 984, AT-B 193 860, AT-B 162 899, DE-B 2 332 277, AT-B 321 311, GB-A 1 590 148).

Es wurde nun gefunden, daß Verbindungen der Formel I

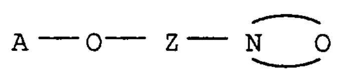

I

worin

A $C_1$-$C_{19}$-Alkyl, Halogen-$C_1$-$C_{12}$-alkyl, Alkenyl mit bis zu 18 C-Atomen, Halogenalkenyl mit bis zu 4 C-Atomen, $C_3$-$C_{12}$-Cycloalkyl, Methylcyclopentyl, Dimethylcyolohexyl, Trimethylcyclohexyl, Tetramethylcyclohexyl, Ethylcyclohexyl, Propyloyolohexyl, Isopropyloyclohexyl, Butyloyclohexyl, Isobutylcyclohexyl, sek.-Butylcyclohexyl, tert.-Butylcyolohexyl, tert.-Amylcyclohexyl, Cyclohexylcyolohexyl, Phenylcyclohexyl, Methylcycloheptyl, Propylcycloheptyl, Cyclopropylmethyl, Cyclopentylmethyl, Cyclohexylmethyl, Cyclohexylethyl, Cyclohexylpropyl, Cycloheptylmethyl, Cyclododecylmethyl, tert.-Butylcyclohexylpropyl, $C_5$-$C_{12}$-Cycloalkenyl, tert.-Butylcyclohexenyl, Cyclohexenylmethyl, tert.-Butylcyclohexenylpropyl, Cyclododecadienylmethyl, Menthyl, Deoalyl, Norbornyl, Tricyclodecanyl, 1,5-Dimethylbicyclo[2,3,1]octan-8-yl, Isobornyl, Adamantyl, Norbornylmethyl, Camphenyl, Homocamphenyl, Pinanyl, Norbornenyl, Nopolyl, Phenyl-$C_1$-$C_6$-alkyl, Halogenphenyl-$C_1$-$C_4$-alkyl, Methylbenzyl, Ethylbenzyl, Propylbenzyl, Butylbenzyl, Methoxybenzyl, Trifluormethylbenzyl, 1-(4-tert.-Butylphenyl)-2-methyl-propyl-3 oder Phenyl-$C_3$-$C_6$-alkenyl,

Z eine Alkylenkette mit 2 bis 10 C-Atomen und

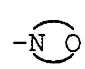

den Rest von 2-Methylmorpholin, 3-Methylmorpholin oder 2,6-Dimethylmorpholin bedeutet,
sowie deren Salze ausgezeichnet wirksam gegen Schadpilze sind und sehr gute Pflanzenverträglichkeit zeigen.

Die neuen Amine der Formel I enthalten ggf. chirale Zentren. Sie werden im allgemeinen als Racemate und gegebenenfalls als Diastereomerengemische erhalten. Einheitliche Diastereomere lassen sich bei einigen der neuen Verbindungen beispielsweise durch Säulenchronatographie oder aufgrund von Löslichkeitsunterschieden in reiner Form isolieren. Aus solchen gereinigten Diastereomeren kann man mit bekannten Methoden einheitliche Racemate und Enantiomere erhalten. Alle diese Verbindungen und Gemische werden von der vorliegenden Erfindung umfaßt. Für die Anwendung der neuen Amine als Fungizide sind sowohl die einheitlichen Diastereomeren bzw. Enantiomeren wie auch deren bei der Synthese anfallenen Gemische geeignet. Bevorzugt werden die letzteren verwendet.

A bedeutet

$C_1$-$C_{19}$-Alkyl wie Methyl, Ethyl, geradkettiges oder verzweigtes Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl,
Halogen-$C_1$-$C_{12}$-alkyl wie Chlorbutyl, Chlorhexyl, Fluorpentyl, Fluorhexyl, Fluorheptyl, Fluordecyl, Fluordodecyl,

Alkenyl mit bis zu 18 C-Atomen wie Allyl, Methallyl, Pentenyl, Hexenyl, Heptenyl, Octenyl, Decenyl, Dodecenyl, Hexadecenyl, Octadecenyl,
Halogenalkenyl mit bis zu 4 C-Atomen wie Chlorallyl, Bromallyl,
$C_3$-$C_{12}$-Cycloalkyl wie Cyclopropyl, Cyclopentyl, Cyclohexyl, Cycloheptyl, Cyclooctyl, Cyclododecyl, Methylcyclopentyl, Dimethylcyclohexyl, Trimethylcyclohexyl, Tetramethylcyclohexyl, Ethylcyclohexyl, Propylcyclohexyl, Iso-Propylcyclohexyl, Butylcyclohexyl, Iso-Butylcyclohexyl, sek.-Butylcyclohexyl, tert.-Butylcyclohexyl, tert.-Amylcyclohexyl, Cyclohexylcyclohexyl, Phenylcyclohexyl, Methylcycloheptyl, Propylcycloheptyl, Cyclopropylmethyl, Cyclopentylmethyl, Cyclohexylmethyl, Cyclohexylethyl, Cyclohexylpropyl, Cycloheptylmethyl, Cyclododecylmethyl, tert.-Butylcyclohexylpropyl,
$C_5$-$C_{12}$-Cycloalkenyl wie Cyclopentenyl, Cycloheptenyl, Cyclooctenyl, Cyclododecadienyl, tert.-Butylcyclohexenyl, Cyclohexenylmethyl, tert.-Butylcyclohexenylpropyl, Cyclododecadienylmethyl, Menthyl, Decalyl, Norbornyl, Tricyclodecanyl, 1,5-Dimethylbicyclo[2,3,1]octan-8-yl, Isobornyl, Adamantyl, Norbornylmethyl, Camphenyl, Homocamphenyl, Pinanyl, Norbornenyl, Nopolyl,
Phenyl-$C_1$-$C_6$-alkyl wie Benzyl, Phenylethyl, Phenylpropyl, Phenylbutyl, Phenylpentyl, Phenylhexyl,
Halogenphenyl-$C_1$-$C_4$-alkyl wie Chlorbenzyl, Fluorbenzyl, Chlorphenylpropyl, Dichlorphenylpropyl, Fluorphenylpropyl,
Methylbenzyl, Ethylbenzyl, Propylbenzyl, Butylbenzyl, Methoxybenzyl, Trifluormethylbenzyl, 1-(4-tert.-Butylphenyl)-2-methyl-propyl-3 oder
Phenyl-$C_3$-$C_6$-alkenyl wie Phenylpropenyl, Phenylbutenyl, Phenylpentenyl.

Die Amine der Formel I lassen sich herstellen, indem man

a) eine Verbindung der Formel II mit einem Amin der Formel III

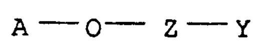 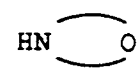

II III

in welcher A und Z die obengenannten Bedeutungen haben und Y für eine nucleophil verdrängbare Abgangsgruppe, beispielsweise Halogen (Cl, Br) oder Alkyl- oder Arylsulfonyl steht, oder

b) ein Alkylierungsmittel der Formel IV mit einem Amin der Formel V

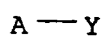 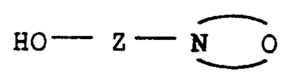

IV V

in welcher A, Z und Y die obengenannten Bedeutungen haben, oder

c) einen Alkohol der Formel VI mit einem Amin der Formel VII

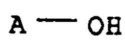 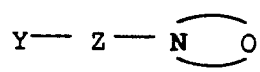

VI VII

in welcher A, Z und Y die oben angegebenen Bedeutungen haben, gegebenenfalls in Gegenwart eines Lösungs- oder Verdünnungsmittels und/oder einer anorganischen oder organischen Base und/oder eines Reaktionsbeschleunigers umsetzt und die so erhaltenen Verbindungen gegebenenfalls in ihre Salze überführt.

Für alle drei Verfabrensvarianten a), b) und c) kommen als Lösungs- oder Verdünnungsmittel beispielsweise Halogenkohlenwasserstoffe, insbesondere Chlorkohlenwasserstoffe, z. B. Tetrachlorethylen-1,1,2,2- oder 1,1,1,2-Tetrachlorethan₃ Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, p-, m-Dibrombenzol, o-, m-, p-Chlortoluol, 1,2,4-Trichlorbenzol; Ether, z. B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, $\beta,\beta'$-Dichlordiethylether; Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z. B. Heptan, Pinan, Nonan, o-, m-, p-Cymol, Benzinfraktionen innerhalb eines Siedepunktintervalles von 70 bis 190 °C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester z. B. Ethylacetat, Acetessigester, Isobutylacetat; Amide, z. B. Formamid, Methylformamid, Dimethylformamid; Ketone, z. B. Aceton, Methylethylketon, gegebenenfalls auch Wasser und entsprechende Gemische in Betracht. Als Lösungsmittel können auch die Verbindungen der Formel III, IV und V im Überschuß verwendet werden. Zweckmäßig verwendet man das Lösungsmittel in einer Menge von 100 bis 2 000 Gew.-%, vorzugsweise von 200 bis 700 Gew.-%, bezogen auf Ausgangsstoff II.

Als anorganische oder organische Basen (Säureakzeptoren) für die Umsetzung zu Verbindungen der Formel I können alle üblichen Säurebindemittel verwendet werden. Hierzu gehören vorzugsweise tertiäre Amine, Erdalkaliverbindungen, Ammoniumverbindungen und Alkaliverbindungen sowie entsprechende Gemische. Es können aber auch Zinkverbindungen verwendet werden. Es kommen z. B. als basische Verbindungen in Frage:

Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Triethylamin, Tripropylamin, Triisopropylamin, Tributylamin, Triisobutylamin, Tri-sec.-butylamin, Tri-tert.-butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-Methylpyrrolidin, N-Ethylpyrrolidon, N-Methylpiperidin, N-Ethylpiperidin, N-Methyl-pyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin,N-Ethylhexamethylenimin, Pyridin, Chinolin, alpha-Picolin, $\beta$-Picolin, gamma-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N-Dimethylcyclohexylamin, 2,6-Lutidin, 2,4-Lutidin, Trifurfurylamin, Triethylendiamin.

Zweckmäßig verwendet man den Säureakzeptor in einem Überschuß oder Unterschuß von bis zu 20 %, bezogen auf den Ausgangsstoff II, IV oder VI.

Als Reaktionsbeschleuniger kommen vorzugsweise Metallhalogenide wie Natriumiodid oder Kaliumiodid in Frage.

Zur Salzbildung mit Verbindungen der Formel I sind alle organischen und anorganischen Säuren geeignet, soweit sie pflanzenphysiologisch verträgliche Salze bilden. So sind z. B. Chloride, Bromide, Jodide, Sulfate, Phosphate, Acetate, Oxalate, Fumarate, Malonate, Alkylsulfonate und Arylsulfonate zu nennen.

Die Salze werden erhalten, indem man die entsprechende Säure mit freiem Amin der Formel I, gegebenenfalls in einem inerten Lösungsmittel zusammengibt, das Lösungsmittel abtrennt und dem Rückstand gegebenefalls umkristallisiert.

Die Ausgangsstoffe der Formel II, in welcher Y für Chlor oder Brom steht, lassen sich nach an sich bekannten Methoden darstellen.

Sie können beispielsweise durch Umsetzung (d) von Alkoholen der Formel IV

A-OH IV

in welcher A die oben angegebene Bedeutung hat, mit $\alpha$,$\omega$-Dihalogenderivaten der Formel VIII

Y-Z-Y VIII

in welcher Z und Y die oben angegebenen Bedeutungen haben, gegebenenfalls in Gegenwart eines oder mehreren Lösungs- und Verdünnungsmitteln und/oder anorganischen Basen und/oder eines Phasentransfer-Katalysators hergestellt werden.

Die Umsetzung (d) wird zweckmäßig in gegenüber der Reaktionsteilnehmern inerten Lösungsmitteln, z. B. Toluol, Xylol, 1,2-Dimethoxyethan, Tetrahydrofuran, Dioxan, Methylenchlorid, Dimethylformamid, Wasser oder deren Gemischen durchgeführt. Als Lösungsmittel können auch die Verbindungen der Formel VIII im Überschuß verwendet werden.

Als säurebindende Mittel kommen z. B. anorganische Basen wie Hydride, Hydroxide, Carbonate, Borate oder Phosphate von Alkali- und Erdalkalimetallen, beispielsweise Natriumhydrid, Natrium- und Kaliumhydroxid, Calciumoxid, Natrium- und Kalium-carbonat und -hydrogencarbonat, Magnesium-, Calcium- oder Bariumcarbonat oder Natrium- und Kaliumphosphate in Frage.

Als Phasentransfer-Katalysatoren kommen vorzugsweise quaternäre Ammonium- und Phosphoniumsalze wie Tetrabutylammonium-chlorid, -hydrogensulfat, -hydroxid, -bromid oder -iodid, Benzyltriethylammoniumchlorid, Cetyltrimethylammoniumchlorid, Benzyltriphenylphosphoniumchlorid oder Kronenether wie 12-Krone-4, 15-Krone-5, 18-Krone-6, Dibenzo-18-krone-6 oder Dicyclohexano-18-krone-6 in Frage.

Die Umsetzungen a), b), c) und d) werden im allgemeinen bei Temperaturen zwischen 0 und 100 °C, drucklos oder unter Druck, kontinuierlich oder diskontinuierlich durchgeführt.

Zwischenprodukte wie z. B. die Alkylierungsreagenzien IV, die Amine III, V und VII und die Alkohole VI sind allgemein bekannt.

Vorschrift 1

Herstellung des Zwischenproduktes

Unter kräftigem Rühren wird eine Mischung aus 130 g (1 Mol) 2-Ethyl-4-methylpentanol-1, 500 ml 1,5-Dichlorpentan, 15 g Tetrabutylammoniumhydrogensulfat und 250 g 50 %iger (Gew.-%), wäßriger Natriumhydroxidlösung 24 Std. auf 50 °C erwärmt. Die Mischung wird sodann mit 1 l Wasser versetzt und viermal mit je 300 ml Methylenchlorid extrahiert. Die vereinigten Extrakte werden fünfmal mit je 200 ml Wasser ausgeschüttelt, über Magnesiumsulfat getrocknet und im Vakuum fraktioniert destilliert.

Man erhält 180 g (77 % d. Th.) 5-(2-Ethyl-4-methylpentoxy)-1-chlorpentanals farblose Flüssigkeit von Sdp. 90 - 92 °C bei 0,3 mbar und $n_D^{22}$ 1,4488.

Auf entsprechende Weise lassen sich die folgenden Zwischenprodukte der Formel A-O-Z-Y herstellen:

| A | Z | Y | Brechungsindex oder Sdp. [°C/mbar] |
|---|---|---|---|
| n-Propyl | $(CH_2)_4$ | Cl | 84 - 86/27 |
| Isopropyl | $(CH_2)_4$ | Cl | 83 - 84/27 |
| n-Butyl | $(CH_2)_4$ | Cl | $n_D^{25}$ = 1,4310 |
| Isobutyl | $(CH_2)_4$ | Cl | 92 - 94/28 |
| n-Butyl | $(CH_2)_6$ | Cl | 98 - 100/27 |
| n-Hexyl | $(CH_2)_4$ | Cl | 122 - 124/30 |
| 1-Chlorhexyl-6 | $(CH_2)_6$ | Cl | $n_D^{22}$ = 1,4485 |
| 2,2-Dimethylpropyl-1 | $(CH_2)_4$ | Cl | 97 - 98/27 |
| 2,2-Dimethylpropyl-1 | $(CH_2)_6$ | Cl | 122 - 125/30 |
| 3,3-Dimethylbutyl-1 | $(CH_2)_4$ | Cl | $n_D^{25}$ = 1,4355 |
| 3,3-Dimethylbutyl-1 | $(CH_2)_6$ | Cl | $n_D^{22}$ = 1,4400 |
| 2,4-Dimethylpentyl-1 | $(CH_2)_4$ | Cl | $n_D^{22}$ = 1,4360 |
| 2,4-Dimethylpentyl-1 | $(CH_2)_6$ | Cl | $n_D^{22}$ = 1,4419 |
| 2-Ethyl-4-methylpentyl-1 | $(CH_2)_3$ | Cl | $n_D^{26}$ = 1,4557 |
| 2-Ethyl-4-methylpentyl-1 | $(CH_2)_4$ | Cl | $n_D^{25}$ = 1,4390 |
| 2-Ethyl-4-methylpentyl-1 | $(CH_2)_6$ | Cl | $n_D^{22}$ = 1,4590 |
| 5-Ethylheptyl-2 | $(CH_2)_4$ | Cl | $n_D^{22}$ = 1,4442 |
| 5-Ethylheptyl-2 | $(CH_2)_6$ | Cl | $n_D^{22}$ = 1,4485 |
| 2-Ethylhexyl-1 | $(CH_2)_3$ | Cl | $n_D^{22}$ = 1,4336 |
| 2-Ethylhexyl-1 | $(CH_2)_4$ | Cl | $n_D^{21}$ = 1,4420 |
| 2-Ethylhexyl-1 | $(CH_2)_5$ | Cl | $n_D^{22}$ = 1,4444 |
| 2-Ethylhexyl-1 | $(CH_2)_6$ | Cl | $n_D^{25}$ = 1,4456 |
| 2-Ethylhexyl-1 | $(CH_2)_8$ | Cl | 133 - 135/0,3 |
| n-Octyl | $(CH_2)_4$ | Cl | $n_D^{21}$ = 1,4418 |
| n-Nonyl | $(CH_2)_4$ | Cl | $n_D^{21}$ = 1,4438 |
| n-Undecyl | $(CH_2)_4$ | Cl | $n_D^{22}$ = 1,4458 |
| 5-Methyl-2-isopropyl-hexyl-1 | $(CH_2)_4$ | Cl | $n_D^{25}$ = 1,4445 |

| A | Z | Y | Brechungsindex oder Sdp. [°C/mbar] |
|---|---|---|---|
| 3-Methylheptyl-2 | $(CH_2)_4$ | Cl | $n_D^{22} = 1,4412$ |
| 3,5,5-Trimethylhexyl-1 | $(CH_2)_4$ | Cl | $n_D^{25} = 1,4430$ |
| 3,7-Dimethyloctyl-1 | $(CH_2)_4$ | Cl | $n_D^{25} = 1,4475$ |
| 3,7-Dimethyloctyl-1 | $(CH_2)_4$ | Cl | $n_D^{25} = 1,4500$ |
| 2-(n-Butyl)-octyl-1 | $(CH_2)_4$ | Cl | $n_D^{25} = 1,4472$ |
| 2-(n-Butyl)-octyl-1 | $(CH_2)_5$ | Cl | $n_D^{22} = 1,4482$ |
| 2-(n-Butyl)-octyl-1 | $(CH_2)_6$ | Cl | $n_D^{22} = 1,4508$ |
| 2-(n-Butyl)-octyl-1 | $(CH_2)_3$ | Cl | $n_D^{22} = 1,4450$ |
| $CF_3CH_2CF_2CH_2$- | $(CH_2)_4$ | Cl | 98 - 100/30 |
| $CF_2H$-$CF_2CH_2$- | $(CH_2)_4$ | Cl | 86 - 88/27 |
| $CF_3(CF_2)_9CH_2CH_2$- | $(CH_2)_4$ | Cl | 116 - 120/0,3 |
| $CF_3(CF_2)_9CH_2CH_2$- | $(CH_2)_4$ | Cl | 125 - 127/0,3 |
| 3,7,11-Trimethyldodecyl-1 | $(CH_2)_4$ | Cl | $n_D^{22} = 1,4523$ |
| 3,7,11-Trimethyldodecyl-1 | $(CH_2)_6$ | Cl | $n_D^{25} = 1,4545$ |
| 2-(n-Hexyl)-decyl-1 | $(CH_2)_4$ | Cl | $n_D^{22} = 1,4520$ |
| 2-(1,3,3-Trimethylbutyl-1)-5,7,7-trimethyloctyl-1 | $(CH_2)_4$ | Cl | $n_D^{22} = 1,4546$ |
| Cyclopropylmethyl | $(CH_2)_6$ | Br | $n_D^{22} = 1,4122$ |
| Cyclopentyl | $(CH_2)_4$ | Cl | $n_D^{20} = 1,4607$ |
| Cyclohexyl | $(CH_2)_4$ | Cl | $n_D^{20} = 1,4660$ |
| Cyclohexylmethyl | $(CH_2)_4$ | Cl | 120 - 122/30 |
| 2-(Cyclohexyl)-propyl-1 | $(CH_2)_4$ | Cl | 106 - 10 8/0,3 |
| 4-tert.-Butylcyclohexyl | $(CH_2)_4$ | Cl | $n_D^{22} = 1,4685$ |
| 4-tert.-Butylcyclohexyl | $(CH_2)_5$ | Cl | $n_D^{25} = 1,4692$ |
| 4-tert.-Butylcyclohexyl | $(CH_2)_6$ | Cl | $n_D^{22} = 1,4675$ |
| Menthyl | $(CH_2)_4$ | Cl | 85 - 90/0,2 |
| Menthyl | $(CH_2)_6$ | Cl | 90 - 92/0,3 |
| Cycloheptyl | $(CH_2)_4$ | Cl | $n_D^{20} = 1,4735$ |

| A | Z | Y | Brechungsindex oder Sdp. [°C/mbar] |
|---|---|---|---|
| 4-tert.-Amylcyclohexyl | $(CH_2)_4$ | Cl | $n_D^{22}$ = 1,4715 |
| 4-tert.-Amylcyclohexyl | $(CH_2)_6$ | Cl | $n_D^{22}$ = 1,4722 |
| Isobornyl | $(CH_2)_4$ | Cl | 88 - 89/0,3 |
| Norbornyl | $(CH_2)_4$ | Cl | $n_D^{22}$ = 1,4775 |
| Norbornyl | $(CH_2)_6$ | Cl | $n_D^{22}$ = 1,4780 |
| Cyclododecyl | $(CH_2)_4$ | Cl | $n_D^{24}$ = 1,4824 |
| Allyl | $(CH_2)_4$ | Cl | 84 - 86/27 |
| Allyl | $(CH_2)_6$ | Cl | 88 - 90/28 |
| Methallyl | $(CH_2)_4$ | Cl | 87 - 89/28 |
| 2-Buten-1-yl | $(CH_2)_4$ | Cl | 87 - 88/28 |
| 3-Methylbut-2-en-1-yl | $(CH_2)_4$ | Cl | 92 - 94/30 |
| Octadec-9-en-1-yl | $(CH_2)_4$ | Cl | $n_D^{21}$ = 1,4625 |
| 3-Chlorallyl | $(CH_2)_4$ | Cl | 87 - 89/27 |
| 2-Chlorallyl | $(CH_2)_4$ | Cl | 80 - 82/28 |
| 2,3,3-Trichlorallyl | $(CH_2)_4$ | Cl | $n_D^{22}$ = 1,5040 |
| 2,3,3-Trichlorallyl | $(CH_2)_6$ | Cl | $n_D^{22}$ = 1,5080 |
| Benzyl | $(CH_2)_4$ | Cl | 118 - 120/28 |
| 4-Methylbenzyl | $(CH_2)_4$ | Cl | 121 - 123/27 |
| 4-Fluorbenzyl | $(CH_2)_4$ | Cl | 117 - 119/30 |
| 4-Chlorbenzyl | $(CH_2)_4$ | Cl | 130 - 132/27 |
| 4-tert.-Butylbenzyl | $(CH_2)_4$ | Cl | 110 - 112/0,2 |
| 4-Methoxybenzyl | $(CH_2)_4$ | Cl | 98 - 99/0,4 |
| 4-Isopropylbenzyl | $(CH_2)_4$ | Cl | 102 - 104/0,2 |
| 3-Phenylpropyl-1 | $(CH_2)_4$ | Cl | $n_D^{22}$ = 1,5043 |
| 3-Phenylpropyl-1 | $(CH_2)_6$ | Cl | $n_D^{25}$ = 1,4995 |
| 3-Phenyl-2-methylpropyl-1 | $(CH_2)_4$ | Cl | $n_D^{22}$ = 1,5005 |
| 3-Phenyl-2-methylpropyl-1 | $(CH_2)_6$ | Cl | $n_D^{25}$ = 1,4961 |

Beispiel

Unter Rühren wird eine Mischung aus 41,1 g (0,15 Mol) 4-(Cyclododecyloxy)-1-chlorbutan, 120 ml cis-2,6-Dimethylmorpholin und 3 g Kaliumiodid 24 Stunden auf 90 °C erwärmt. Nach Abkühlen auf 10 °C wird das Reaktionsgemisch nacheinander mit 100 ml Petrolether und 70 ml 20 %iger wäßriger Natriumhydroxidlösung versetzt. Die organische Phase wird dreimal mit je 80 ml Wasser gewaschen, über $Na_2SO_4$ getrocknet und im Vakuum fraktioniert destilliert.

Man erhält 46,8 g (88,4 % d. Th.) N-(Cyclododecyloxy-butyl)-cis-2,6-dimethyl-morpholin als farbloses Öl vom Sdp. 181 - 182 °C/0,15 mbar und $n_D^{22}$ = 1,4832.

In analoger Weise können die nachstehend aufgelasteten Verbindungen der Formel I erhalten werden.

| Beispiel Nr. | A | Z | 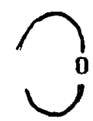 | Brechungsindex oder Sdp. [°C/mbar] |
|---|---|---|---|---|
| 2 | n-Butyl | $(CH_2)_4$ | $-CH_2-CH(CH_3)OCH(CH_3)CH_2-$ | $n_D^{25}$ = 1,4485 |
| 3 | n-Hexyl | $(CH_2)_4$ | $-CH_2-CH(CH_3)OCH(CH_3)CH_2-$ | $n_D^{25}$ = 1,4505 |
| 4 | 2,2-Dimethylprop-1-yl | $(CH_2)_6$ | $-CH_2-CH(CH_3)OCH(CH_3)CH_2-$ | $n_D^{22}$ = 1,4465 |
| 5 | 2,2-Dimethylprop-1-yl | $(CH_2)_6$ | $-CH_2-CH(CH_3)OCH(CH_3)CH_2-$ | |
| 6 | 3,3-Dimethylbut-1-yl | $(CH_2)_4$ | $-CH_2-CH(CH_3)OCH(CH_3)CH_2-$ | $n_D^{22}$ = 1,4490 |
| 7 | 3,3-Dimethylbut-1-yl | $(CH_2)_6$ | $-CH_2-CH(CH_3)OCH(CH_3)CH_2-$ | $n_D^{22}$ = 1,4510 |
| 8 | 2,4-Dimethylpent-1-yl | $(CH_2)_4$ | $-CH_2-CH(CH_3)OCH(CH_3)CH_2-$ | $n_D^{22}$ = 1,4511 |
| 9 | 2,4-Dimethylpent-1-yl | $(CH_2)_6$ | $-CH_2-CH(CH_3)OCH(CH_3)CH_2-$ | $n_D^{22}$ = 1,4520 |
| 10 | 2-Ethyl-4-methylpent-1-yl | $(CH_2)_3$ | $-CH_2-CH(CH_3)OCH(CH_3)CH_2-$ | $n_D^{22}$ = 1,4502 |

| Beispiel Nr. | A | Z | 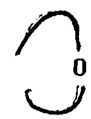 | Brechungsindex oder Sdp. [°C/mbar] |
|---|---|---|---|---|
| 11 | 2-Ethyl-4-methylpent-1-yl | $(CH_2)_4$ | $-CH_2-CH(CH_3)OCH(CH_3)CH_2-$ | $n_D^{25}$ = 1,4510 |
| 12 | 2-Ethyl-4-methylpent-1-yl | $(CH_2)_5$ | $-CH_2-CH(CH_3)OCH(CH_3)CH_2-$ | $n_D^{22}$ = 1,4532 |
| 13 | 2-Ethyl-4-methylpent-1-yl | $(CH_2)_6$ | $-CH_2-CH(CH_3)OCH(CH_3)CH_2-$ | 141 - 143/0,3 |
| 14 | n-Octyl | $(CH_2)_4$ | $-CH_2CH(CH_3)OCH(CH_3)CH_2-$ | $n_D^{22}$ = 1,4519 |
| 15 | n-Nonyl | $(CH_2)_4$ | $-CH_2-CH(CH_3)O-CH(CH_3)CH_2-$ | $n_D^{25}$ = 1,4533 |
| 16 | 5-Ethylhept-2-yl | $(CH_2)_4$ | $-CH_2-CH(CH_3)O-CH(CH_3)CH_2-$ | $n_D^{22}$ = 1,4534 |
| 17 | 5-Ethylhept-2-yl | $(CH_2)_6$ | $-CH_2-CH(CH_3)O-CH(CH_3)CH_2-$ | $n_D^{22}$ = 1,4577 |

| Beispiel Nr. | A | Z | 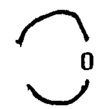 | Brechungsindex oder Sdp. [°C/mbar] |
|---|---|---|---|---|
| 18 | 3,5,5-Trimethylhex-1-yl | $(CH_2)_4$ | $-CH_2-CH(CH_3)O-CH(CH_3)CH_2-$ | $n_D^{22}$ = 1,4530 |
| 19 | 3,5,5-Trimethylhex-1-yl | $(CH_2)_4$ | $-CH_2-CH(CH_3)O-CH(CH_3)CH_2-$ | $n_D^{22}$ = 1,4545 |
| 20 | 2-Ethylhex-1-yl | $(CH_2)_3$ | $-CH_2CH(CH_3)OCH(CH_3)CH_2-$ | $n_D^{22}$ = 1,4507 |
| 21 | 2-Ethylhex-1-yl | $(CH_2)_4$ | $-CH_2CH(CH_3)OCH(CH_3)CH_2-$ | $n_D^{22}$ = 1,4525 |
| 22 | 2-Ethylhex-1-yl | $(CH_2)_5$ | $-CH_2CH(CH_3)OCH(CH_3)CH_2-$ | $n_D^{22}$ = 1,4528 |
| 23 | 2-Ethylhex-1-yl | $(CH_2)_6$ | $-CH_2CH(CH_3)OCH(CH_3)CH_2-$ | $n_D^{22}$ = 1,4549 |
| 24 | 5-Methyl-2-isopropylhex-1-yl | $(CH_2)_4$ | $-CH_2CH(CH_3)OCH(CH_3)CH_2-$ | $n_D^{25}$ = 1,4540 |
| 25 | 3,7-Dimethyloct-1-yl | $(CH_2)_4$ | $-CH_2CH(CH_3)OCH(CH_3)CH_2-$ | $n_D^{25}$ = 1,4545 |

| Beispiel Nr. | A | Z | 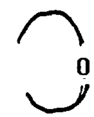 | Brechungsindex oder Sdp. [°C/mbar] |
|---|---|---|---|---|
| 26 | 3,7-Dimethyloct-1-yl | $(CH_2)_6$ | $-CH_2CH(CH_3)OCH(CH_3)CH_2-$ | $n_D^{25}$ = 1,4565 |
| 27 | 3,7-Dimethyloct-1-yl | $(CH_2)_4$ | $-CH_2CH(CH_3)OCH(CH_3)CH_2-$ | 167 - 169/0,4 |
| 28 | n-Decyl | $(CH_2)_3$ | $-CH_2CH(CH_3)OCH(CH_3)CH_2-$ | $n_D^{23}$ = 1,4781 |
| 29 | 2-(n-Butyl)-oct-1-yl | $(CH_2)_3$ | $-CH_2CH(CH_3)OCH(CH_3)CH_2-$ | $n_D^{23}$ = 1,4555 |
| 30 | 2-(n-Butyl)-oct-1-yl | $(CH_2)_4$ | $-CH_2CH(CH_3)OCH(CH_3)CH_2-$ | $n_D^{25}$ = 1,4550 |
| 31 | 2-(n-Butyl)-oct-1-yl | $(CH_2)_5$ | $-CH_2CH(CH_3)OCH(CH_3)CH_2-$ | $n_D^{22}$ = 1,4569 |
| 32 | 2-(n-Butyl)-oct-1-yl | $(CH_2)_6$ | $-CH_2CH(CH_3)OCH(CH_3)CH_2-$ | $n_D^{22}$ = 1,4570 |
| 33 | 3-Methylhept-1-yl | $(CH_2)_4$ | $-CH_2-CH(CH_3)OCH(CH_3)CH_2-$ | $n_D^{22}$ = 1,4538 |
| 34 | n-Undecyl | $(CH_2)_4$ | $-CH_2-CH(CH_3)OCH(CH_3)CH_2-$ | $n_D^{22}$ = 1,4558 |
| 35 | 2-(n-Hexyl)-dec-1-yl | $(CH_2)_4$ | $-CH_2-CH(CH_3)OCH(CH_3)CH_2-$ | $n_D^{26}$ = 1,4562 |
| 36 | 3,7,11-Trimethyldodec-1-yl | $(CH_2)_4$ | $-CH_2-CH(CH_3)OCH(CH_3)CH_2-$ | $n_D^{26}$ = 1,4557 |
| 37 | 2-(n-Hexyl)-dec-1-yl | $(CH_2)_4$ | $-CH_2-CH(CH_3)OCH(CH_3)CH_2-$ | 200 - 202/0,6 |

| Beispiel Nr. | A | Z | 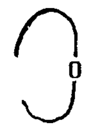 | Brechungsindex oder Sdp. [°C/mbar] |
|---|---|---|---|---|
| 38 | 2-(1,3,3-Trimethylbutyl-1)--5,7,7-trimethyloct-1-yl | $(CH_2)_4$ | $-CH_2-CH(CH_3)OCH(CH_3)CH_2-$ | $n_D^{26}$ = 1,4581 |
| 39 | 8-Chloroct-1-yl | $(CH_2)_4$ | $-CH_2-CH(CH_3)OCH(CH_3)CH_2-$ | 133 - 135/0,3 |
| 40 | $CF_3CF_2CF_2CH_2-$ | $(CH_2)_4$ | $-CH_2-CH(CH_3)OCH(CH_3)CH_2-$ | |
| 41 | $CF_3CF_2CF_2CH_2-$ | $(CH_2)_6$ | $-CH_2-CH(CH_3)OCH(CH_3)CH_2-$ | |
| 42 | $CF_3\ (CF_2)_9-CH_2-CH_2-$ | $(CH_2)_4$ | $-CH_2-CH(CH_3)OCH(CH_3)CH_2-$ | $n_D^{25}$ = 1,4335 |
| 43 | Cyclopentyl | $(CH_2)_4$ | $-CH_2-CH(CH_3)OCH(CH_3)CH_2-$ | $n_D^{22}$ = 1,4655 |
| 44 | Cyclohexyl | $(CH_2)_4$ | $-CH_2-CH(CH_3)OCH(CH_3)CH_2-$ | $n_D^{22}$ = 1,4639 |
| 45 | Cyclohexyl | $(CH_2)_6$ | $-CH_2-CH(CH_3)OCH(CH_3)CH_2-$ | $n_D^{22}$ = 1,4643 |
| 46 | Cyclohexylmethyl | $(CH_2)_4$ | $-CH_2-CH(CH_3)OCH(CH_3)CH_2-$ | 136 - 140/0,4 |
| 47 | (4-tert.-Butyl)-cyclohexyl | $(CH_2)_4$ | $-CH_2-CH(CH_3)OCH(CH_3)CH_2-$ | $n_D^{22}$ = 1,4716 |
| 48 | (4-tert.-Butyl)-cyclohexyl | $(CH_2)_6$ | $-CH_2-CH(CH_3)OCH(CH_3)CH_2-$ | $n_D^{22}$ = 1,4710 |
| 49 | (4-tert.-Amyl)-cyclohexyl | $(CH_2)_4$ | $-CH_2-CH(CH_3)OCH(CH_3)CH_2-$ | $n_D^{22}$ = 1,4751 |
| 50 | (4-tert.-Amyl)-cyclohexyl | $(CH_2)_6$ | $-CH_2-CH(CH_3)OCH(CH_3)CH_2-$ | $n_D^{22}$ = 1,4745 |

168 - 170/0,4

| Beispiel Nr. | A | Z | 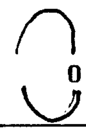 | Brechungsindex oder Sdp. [°C/mbar] |
|---|---|---|---|---|
| 51 | 3,5,5-Dimethylcyclohexyl | $(CH_2)_4$ | $-CH_2-CH(CH_3)OCH(CH_3)CH_2-$ | $n_D^{22}$ = 1,4672 |
| 52 | 3,5,5-Dimethylcyclohexyl | $(CH_2)_5$ | $-CH_2-CH(CH_3)OCH(CH_3)CH_2-$ | |
| 53 | 3,5,5-Dimethylcyclohexyl | $(CH_2)_6$ | $-CH_2-CH(CH_3)OCH(CH_3)CH_2-$ | |
| 54 | 2-(Cyclohexyl)-prop-1-yl | $(CH_2)_4$ | $-CH_2-CH(CH_3)OCH(CH_3)CH_2-$ | $n_D^{22}$ = 1,4731 |
| 55 | 2-(Cyclohexyl)-prop-1-yl | $(CH_2)_6$ | $-CH_2-CH(CH_3)OCH(CH_3)CH_2-$ | 140 - 142/0,4 |
| 56 | 4-(Cyclohexyl)-cyclohexyl | $(CH_2)_4$ | $-CH_2-CH(CH_3)OCH(CH_3)CH_2-$ | $n_D^{22}$ = 1,4871 |
| 57 | 4-(Cyclohexyl)-cyclohexyl | $(CH_2)_6$ | $-CH_2-CH(CH_3)OCH(CH_3)CH_2-$ | |
| 58 | Cycloheptyl | $(CH_2)_4$ | $-CH_2-CH(CH_3)OCH(CH_3)CH_2-$ | $n_D^{22}$ = 1,4740 |
| 59 | 1-Norbornyl | $(CH_2)_4$ | $-CH_2-CH(CH_3)OCH(CH_3)CH_2-$ | $n_D^{22}$ = 1,4784 |
| 60 | 1-Norbornyl | $(CH_2)_6$ | $-CH_2-CH(CH_3)OCH(CH_3)CH_2-$ | $n_D^{22}$ = 1,4780 |

| Beispiel Nr. | A | Z | 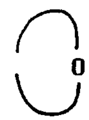 | Brechungsindex oder Sdp. [°C/mbar] |
|---|---|---|---|---|
| 61 | 1-Decalyl | $(CH_2)_4$ | $-CH_2-CH(CH_3)OCH(CH_3)CH_2-$ | |
| 62 | 2-Decalyl | $(CH_2)_4$ | $-CH_2-CH(CH_3)OCH(CH_3)CH_2-$ | |
| 63 | Isobornyl | $(CH_2)_4$ | $-CH_2-CH(CH_3)OCH(CH_3)CH_2-$ | |
| 64 | Allyl | $(CH_2)_6$ | $-CH_2-CH(CH_3)OCH(CH_3)CH_2-$ | 125 - 118/0,3 |
| 65 | Methallyl | $(CH_2)_6$ | $-CH_2-CH(CH_3)OCH(CH_3)CH_2-$ | 120 - 121/0,3 |
| 66 | 2,3,3-Trichlorallyl | $(CH_2)_4$ | $-CH_2-CH(CH_3)OCH(CH_3)CH_2-$ | $n_D^{22}$ = 1,4940 |
| 67 | 2,3,3-Trichlorallyl | $(CH_2)_6$ | $-CH_2-CH(CH_3)OCH(CH_3)CH_2-$ | $n_D^{22}$ = 1,4875 |
| 68 | Benzyl | $(CH_2)_2$ | $-CH_2-CH(CH_3)OCH(CH_3)CH_2-$ | 135 - 138/0,2 |

| Beispiel Nr. | A | Z | 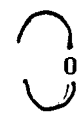 | Brechungsindex oder Sdp. [°C/mbar] |
|---|---|---|---|---|
| 69 | 2,6-Dichlorbenzyl | $(CH_2)_2$ | $-CH_2-CH(CH_3)OCH(CH_3)CH_2-$ | 158 - 163/0,4 |
| 70 | 2,6-Dichlorbenzyl | $(CH_2)_2$ | $-CH_2-CH(CH_3)OCH(CH_3)CH_2-$ | 155 - 158/0,5 |
| 71 | 4-Tert.-Butylbenzyl | $(CH_2)_2$ | $-CH_2-CH(CH_3)OCH(CH_3)CH_2-$ | 156/0,1 |
| 72 | 3-Phenyl-prop-1-yl | $(CH_2)_4$ | $-CH_2-CH(CH_3)OCH(CH_3)CH_2-$ | $n_D^{22}$ = 1,4978 |
| 73 | 3-Phenyl-prop-1-yl | $(CH_2)_6$ | $-CH_2-CH(CH_3)OCH(CH_3)CH_2-$ | $n_D^{22}$ = 1,4925 |
| 74 | 3-Phenyl-prop-1-yl | $(CH_2)_5$ | $-CH_2-CH(CH_3)OCH(CH_3)CH_2-$ | $n_D^{21}$ = 1,4931 |
| 75 | 3-Phenyl-2-methylprop-1-yl | $(CH_2)_4$ | $-CH_2-CH(CH_3)OCH(CH_3)CH_2-$ | $n_D^{22}$ = 1,4953 |
| 76 | 3-Phenyl-2-methylprop-1-yl | $(CH_2)_6$ | $-CH_2-CH(CH_3)OCH(CH_3)CH_2-$ | $n_D^{22}$ = 1,4930 |
| 77 | 3-(4-tert.-Butylphenyl)-2-methylprop-1-yl | $(CH_2)_4$ | $-CH_2-CH(CH_3)OCH(CH_3)CH_2-$ | 179 - 180/0,2 |
| 78 | 3-(4-tert.-Butylphenyl)-2-methylprop-1-yl | $(CH_2)_5$ | $-CH_2-CH(CH_3)OCH(CH_3)CH_2-$ | $n_D^{22}$ = 1,4922 |

| Beispiel Nr. | A | Z | 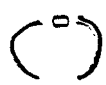 | Brechungsindex oder Sdp. [°C/mbar] |
|---|---|---|---|---|
| 79 | 3-(4-tert.-Butylphenyl)-2-methylprop-1-yl | $(CH_2)_6$ | $-CH_2-CH(CH_3)OCH(CH_3)CH_2-$ | $n_D^{22} = 1,4915$ |
| 80 | 3-(4-tert.-Butylphenyl)-2-methylprop-1-yl | $(CH_2)_8$ | $-CH_2-CH(CH_3)OCH(CH_3)CH_2-$ | $n_D^{22} = 1,4909$ |
| 81 | 3-(4-tert.-Butylphenyl)-2-methylprop-1-yl | $(CH_2)_4$ | $-CH_2-CH(CH_3)OCH(CH_3)CH_2-$ | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten,-aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, So ja, Kaffee, Zuckerrohr, Obst und Zierpflanzen im Gartenbau, Weinbau sowie Gemüse, wie Gurken, Bohnen und Kürbisgewächse.

Die neuen Verbindungen sind insbesondere geeignet zur Bekämpfung folgender Pflanzenkrankheiten:
Erysiphe graminis an Getreide,
Erysiphe cichoracearum bzw. Sphaerotheca fuliginea an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Pyrenophora teres an Gerste
Botrytis cinerea (Grauschimmel) an Erdbeeren, Reben,
Cercospora musae an Bananen,
Pseudocercosporella herpotrichoides an Weizen, Gerste,
Pyricularia oryzae an Reis,
Hemileia vastatrix an Kaffee,
Alternaria solani an Kartoffeln, Tomaten
Plasmopara viticola an Reben sowie Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle, (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung Nr. 3 mit 10 Gewichtsteilen N-Methyl-$\alpha$-pyrrolidon und erhält eine Lösung, die zur Anwendung in Form kleinster Tropfen geeignet ist.

II. 20 Gewichtsteile der Verbindung Nr. 9 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung Nr. 10 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung Nr. 11 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 12 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung Nr. 16 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung Nr. 20 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 22 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 31 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensatesund 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindrnngsgemaßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid
Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,
heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino-1-(bis-(dimethylamino)-phosphinyl)-3-phenyl-1,2,4-triazol
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester

2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethylthio-phthalimid
N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Iod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol
5-Butyl-2-dimethylamino-4-hyroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
sowie verschiedene Fungizide, wie
Dodecylguanidinacetat
3-(3-(3,5-Dimethyl-2-oxycyclohexyl)-2-hydroxyethyl)-gluataramid Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl(-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid.
2-Cyano-N-(ethylaminocarbonyl)-2-methoximino)-acetamid
1-(2-(2,4-Dichlorphenyl)-pentyl)-1H-1,2,4-triazol
2,4-Difluor-alpha-(1H-1,2,4-triazolyl-1-methyl)-benzhydrylalkohol

Für die folgenden Versuche wurden als Vergleich die bekannten Wirkstoffe N-Tridecyl-2,6-dimethylmorpholin (A) und sein Acetat (B) verwendet.

Anwendungsbeispiel 1

Wirksamkeit gegen Weizenmehltau

Blätter von Töpfen gewachsenen Weizenkeimlingen der Sorte "Frühgold" werden mit wäßriger Spritzbrühe, die 80 % Wirkstoff und 20 % Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 7 Tagen wird das Ansmaß der Mehltauentwicklung ermittelt.

Das Ergebnis zeigt, daß die Verbindungen 3, 9, 10, 11, 12, 16, 20, 22, 31, 36, 49, 51, 67, 78, 80 bei der Anwendung als 0,025 oder 0,006%ige Wirkstoffbrühe eine bessere fungizide Wirkung (etwa 97 %) zeigen als die bekannten Vergleichsmittel A und B (etwa 90 %).

Anwendungsbeispiel 2

Wirksamkeit gegen Gurkenmehltau (kurativ)

Junge Gurkenpflanzen der Sorte "Chinesische Schlange" werden im Zweiblattstadium mit einer wäßrigen Konidiensuspension des Gurkenmehltaus (Erysiphe cichoracearum und Sphaerotheca fuliginea) besprüht. Nach 20 Stunden werden diese Pflanzen mit wäßriger Spritzbrühe, die 80 % Wirkstoff und 20 % Emulgiermittel in der Trockensubstanz enthält, bis zur Tropfnässe besprüht und im Gewächshaus bei Temperaturen zwischen 20 und 22°C und 70 bis 80 % Luftfeuchtigkeit aufgestellt. 21 Tage nach der Wirkstoffapplikation wird das Ausmaß des Pilzbefalls ermittelt.

Das Ergebnis zeigt, daß die Verbindungen 1, 10, 22, 26, 29, 31, 32, 60, 73, 78 bei der Anwendung als 0,025%ige Wirkstoffbrühe eine bessere fungizide Wirkung zeigen (etwa 97 %) als der bekannte Vergleichswirkstoff A (etwa 60 %).

Anwendungsbeispiel 3

Wirksamkeit gegen Weizenbraunrost

Blätter von in Töpfen gewachsenen Weizensämlingen der Sorte "Frühgold" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel in der Trockensubstanz enthalten, tropfnaß gespritzt. Nach dem Antrocknen des Spritzbelages werden die Versuchspflanzen im Gewächshaus bei Temperaturen zwichen 20 und 22°C und 65 bis 70 % relativer Luftfeuchte aufgestellt. Nach 8 Tagen wird das Ausmaß der Rostplizentwicklung auf den Blättern ermittelt.

Das Ergebnis zeigt, daß die Verbindungen 1, 8, 11, 17, 28, 69, 72 bei der Anwendung als 0,025%ige Wirkstoffbrühe eine bessere fungizide Wirkung zeigen (etwa 90 %) als der bekannte Vergleichswirkstoff A (etwa 50 %).

Anwendungsbeispiel 4

Wirksamkeit gegen Pyrenophora teres

Blätter von in Töpfen gewachsenen Gerstenkeimlingen der Sorte "Asse" werden im Zweiblattstadium mit wäßriger Spritzbrühe, die 80 % Wirkstoff und 20 % Emulgiermittel in der Trockensubstanz enthält, bis zur Tropfnässe besprüht. Am folgenden Tage werden die angetrockneten Pflanzen mit einer wäßrigen Sporensuspension von Pyrenophora teres inoculiert und für 7 Tage bei 17 bis 19°C und 95 % relativer Luftfeuchigkeit weiter kultiviert. Dann wird das Ausmaß des Pilzbefalls ermittelt.

Das Ergebnis zeigt, daß die Wirkstoffe 9, 16, 43, 47, 58, 59, 60, 66 bei der Anwendung als 0,025%ige Wirkstoffbrühe eine gute fungizide Wirkung zeigen (etwa 90 %).

## Patentansprüche

**1.** Amine der allgemeinen Formel I

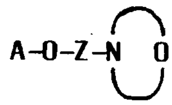 I

worin

A $C_1$-$C_{19}$-Alkyl, Halogen-$C_1$-$C_{12}$-alkyl, Alkenyl mit bis zu 18 C-Atomen, Halogenalkenyl mit bis zu 4 C-Atomen, $C_3$-$C_{12}$-Cycloalkyl, Methylcyclopentyl, Dimethylcyclohexyl, Trimethylcyclohexyl, Tetramethylcyclohexyl, Ethylcyclohexyl, Propylcyclohexyl, Isopropylcyclohexyl, Butylcyclohexyl, Isobutylcyclohexyl, sek.-Butylcyclohexyl, tert.-Butylcyclohexyl, tert.-Amylcyclohexyl, Cyclohexylcyclohexyl, Phenylcyclohexyl, Methylcycloheptyl, Propylcycloheptyl, Cyclopropylmethyl, Cyclopentylmethyl, Cyclohexylmethyl, Cyclohexylethyl, Cyclohexylpropyl, Cycloheptylmethyl, Cyclododecylmethyl, tert.-Butylcyclohexylpropyl, $C_5$-$C_{12}$-Cycloalkenyl, Cyclohexenylmethyl, tert.-Butylcyclohexenyl, tert.-Butylcyclohexenylpropyl Cyclododecadienylmethyl, Menthyl, Decalyl, Norbornyl, Tricyclodecanyl, 1,5-Dimethylbicyclo[2,3,1]octan-8-yl, Isobornyl, Adamantyl, Norbornylmethyl, Camphenyl, Homocamphenyl, Pinanyl, Norbornenyl, Nopolyl, Phenyl-$C_1$-$C_6$-alkyl, Halogenphenyl-$C_1$-$C_4$-alkyl, Methylbenzyl, Ethylbenzyl, Propylbenzyl, Butylbenzyl, Methoxybenzyl, Trifluormethylbenzyl, 1-(4-tert.-Butylphenyl)-2-methyl-propyl-3 oder Phenyl-$C_3$-$C_6$-alkenyl bedeutet,

Z eine Alkylenkette mit 2 bis 10 C-Atomen bedeutet und

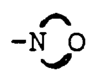

den Rest von 2-Methylmorpholin, 3-Methylmorpholin oder 2,6-Dimethylmorpholin bedeutet und deren Salze.

**2.** Verfahren zur Herstellung von Aminen gemäß Anspruch 1, dadurch gekennzeichnet, daß man

a) eine Verbindung der Formel II mit einem Amin der Formel III

A-O-Z-Y II

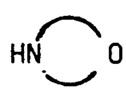

III

in welcher A, O, Z und

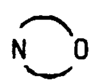

die oben genannten Bedeutungen haben und Y für eine nucleophil verdrängbare Abgangsgruppe steht, oder

b) ein Alkylierungsmittel der Formel IV mit einem Amin der Formel V

A-Y IV

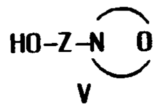

V

in welcher A, Z, Y und

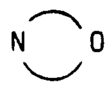

die obengenannten Bedeutungen haben, oder
c) einen Alkohol der Formel VI mit einem Amin der Formel VII

A-OH VI

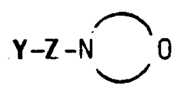

VII

in welcher A, Z, Y und

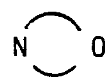

die oben genannten Bedeutungen haben, umsetzt und die erhaltenen Verbindungen gegebenenfalls in ihre Salze überführt.

**3.** Fungizid, enthaltend einen festen oder flüssigen Trägerstoff und ein Amin der allgemeinen Formel I gemäß Anspruch 1.

**4.** Verfahren zur Herstellung eines Fungizids, dadurch gekennzeichnet, daß man einen festen oder flüssigen Trägerstoff mit einem Amin der allgemeinen Formel I gemäß Anspruch 1 vermischt.

**5.** Verfahren zur Bekämpfung von Pilzen, dadurch gekennzeichnet, daß man die Pilze oder die vor Pilzbefall zu schützenden Materialien, Pflanzen, Böden oder Saatgüter mit einer fungizid wirksamen Menge eines Amins der allgemeinen Formel I gemäß Anspruch 1 behandelt.

**Claims**

**1.** An amine of the general formula I

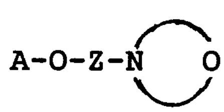

I

wherein

A is $C_1$-$C_{19}$-alkyl, halo-$C_1$-$C_{12}$-alkyl, alkenyl having up to 18 C atoms, haloalkenyl having up to 4 C atoms, $C_3$-$C_{12}$-cycloalkyl, methylcyclopentyl, dimethylcyclohexyl, trimethylcyclohexyl, tetramethylcyclohexyl, ethylcyclohexyl, propylcyclohexyl, isopropylcyclohexyl, butylcyclohexyl, isobutylcyclohexyl, sec-butylcyclohexyl, tert-butylcyclohexyl, tert-amylcyclohexyl, cyclohexylcyclohexyl, phenylcyclohexyl, methylcycloheptyl, propylcycloheptyl, cyclopropylmethyl, cyclopentylmethyl, cyclohexylmethyl, cyclohexylethyl, cyclohexylpropyl, cycloheptylmethyl,

cyclododecylmethyl, tertbutylcyclohexylpropyl, $C_5$-$C_{12}$-cycloalkenyl, cyclohexenylmethyl, tert-butylcyclohexenyl, tert-butylcyclohexenylpropyl, cyclododecadienylmethyl, menthyl, decalyl, norbornyl, tricyclodecanyl, 1,5-dimethylbicyclo[2,3,1]octan-8-yl, isobornyl, adamantyl, norbornylmethyl, camphenyl, homocamphenyl, pinanyl, norbornenyl, nopolyl, phenyl-$C_1$-$C_6$-alkyl, halophenyl-$C_1$-$C_4$-alkyl, methylbenzyl, ethylbenzyl, propylbenzyl, butylbenzyl, methoxybenzyl, trifluoromethylbenzyl, 1-(4-tert-butylphenyl)-2-methyl-propyl-3 or phenyl-$C_3$-$C_6$-alkenyl,

Z is an alkylene chain having 2 to 10 C atoms, and

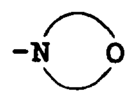

is the radical of 2-methylmorpholine, 3-methylmorpholine or 2,6-dimethylmorpholine or its salts.

**2.** A process for preparing amines as claimed in Claim 1, which comprises reacting

a) a compound of the formula II with an amine of the formula III

A-O-Z-Y II

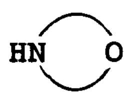

III

in which A, O, Z and

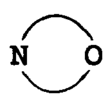

have the above-mentioned meanings and Y is a nucleophillically displaceable leaving group, or

b) an alkylating agent of the formula IV with an amine of the formula V

A-Y IV

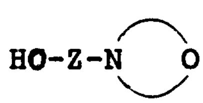

V

in which A, Z, Y and

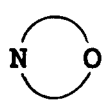

have the above-mentioned meanings, or

c) an alcohol of the formula VI with an amine of the formula VII

A-OH VI

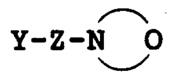

VII

in which A, Z, Y and

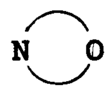

have the above-mentioned meanings, and if desired converting the compounds obtained into their salts.

**3.** A fungicide containing a solid or liquid carrier and an amine of the general formula I as claimed in Claim 1.

**4.** A process for preparing a fungicide, which comprises mixing a solid or liquid carrier with an amine of the general formula I as claimed in Claim 1.

**5.** A method of controlling fungi, which comprises treating the fungi or the materials, plants, soil or seed to be protected from fungal attack with a fungicidally active amount of an amine of the general formula I as claimed in Claim 1.

**Revendications**

**1.** Amines de formule générale I

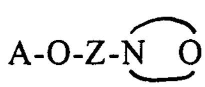

I

dans laquelle
A représente alkyle en C1-C19, halogénalkyle en C1-C12, alcényle ayant jusqu'à 18 atomes C, halogénalcényle ayant jusqu'à 4 atomes C, cycloalkyle en C3-C12, méthylcyclopentyle, diméthylcyclohexyle, triméthylcyclohexyle, tétraméthylcyclohexyle, éthylcyclohexyle, propylcyclohexyle, isopropylcyclohexyle, butylcyclohexyle, isobutylcyclohexyle, sec-butylcyclohexyle, ter-butylcyclohexyle, teramylcyclohexyle, cyclohexylcyclohexyle, phénylcyclohexyle, méthylcycloheptyle, propylcycloheptyle, cyclopropylméthyle, cyclopentylméthyle, cyclohexylméthyle, cyclohexyléthyle, cyclohexylpropyle, cycloheptylméthyle, cyclododécylméthyle, terbutylcyclohexylpropyle, cycloalcényle en C5-C12, cyclohexénylméthyle, ter-butylcyclohexényle, ter-butylcyclohexénylpropyle, cyclododécadiénylméthyle, menthyle, décalyle, norbornyle, tricyclodécanyle, 1,5-diméthylbicyclo[2,3,1]octan-8-yle, isobornyle, adamantyle, norbornylméthyle, camphényle, homocamphényle, pinanyle, norbornényle, nopolyle, phényl-alkyle en C1-C6, halogénophényl-alkyle en C1-C4, méthylbenzyle, éthylbenzyle, propylbenzyle, butylbenzyle, méthoxybenzyle, trifluorométhylbenzyle, 1-(4-ter-butylphényl)-2-méthylpropyle-3 ou phényl-alcényle en C3-C8,
Z représente une chaîne alkylène de 2 à 10 atomes C, et

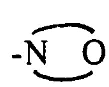

est le reste de 2-méthylmorpholine, 3-méthylmorpholine ou 2,6-diméthylmorpholine,
ainsi que leurs sels.

**2.** Procédé de préparation d'amines selon la revendication 1, caractérisé par le fait que l'on fait réagir

a) un composé de formule II avec un amine de formule III

A-O-Z-Y II

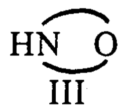

III

dans laquelle A, O, Z et

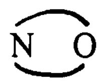

ont les significations données plus haut et Y est mis pour un groupe éliminable par un réactif nucléophile,

b) un agent d'alkylation de formule IV avec une amine de formule V

A-Y IV

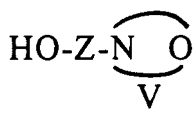

V

dans laquelle A, Z, Y et

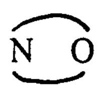

ont les significations données plus haut, ou

c) un alcool de formule VI avec une amine de formule VII

A-OH VI

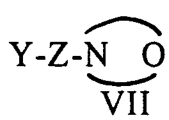

VII

dans laquelle A, Z, Y et

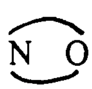

ont les significations données plus haut et on transforme éventuellement les composés obtenus en leurs sels.

**3.** Fongicide, contenant un support solide ou liquide et une amine de formule générale I selon la revendication 1.

**4.** Procédé de préparation d'un fongicide, caractérisé par le fait que l'on mélange un support solide ou liquide avec une amine de formule générale I selon la revendication 1.

**5.** Procédé de lutte contre les champignons, caractérisé par le fait que l'on traite les champignons ou les matériaux, plantes, sols ou semences à protéger d'une attaque des champignons avec une quantité efficace au point de vue fongicide d'une amine de formule générale I selon la revendication 1.